# EUROPEAN PATENT SPECIFICATION

(11) **EP 2 069 518 B1**
(45) Date of publication and mention of the grant of the patent: **03.02.2010**
(21) Application number: 07818125.2
(22) Date of filing: 12.09.2007
(51) Int. Cl.: C12P 13/22

(54) **METHOD FOR PRODUCING OPTICALLY ACTIVE PHENYLALANINE COMPOUNDS FROM CINNAMIC ACID DERIVATIVES EMPLOYING A PHENYLALANINE AMMONIA LYASE DERIVED FROM IDIOMARINA LOIHIENSIS**
VERFAHREN ZUR HERSTELLUNG OPTISCH AKTIVER PHENYLALANINVERBINDUNGEN AUS ZIMTSÄUREDERIVATEN MIT EINER PHENYLALANINAMMONIUMLYASE AUS IDIOMARINA LOIHIENSIS
PROCÉDÉ DE PRODUCTION DE COMPOSÉS DE PHÉNYLALANINE OPTIQUEMENT ACTIFS À PARTIR DE DÉRIVÉS D'ACIDE CINNAMIQUE UTILISANT UNE PHENYLALANINE AMMONIA LYASE DÉRIVÉE D'IDIOMARINA LOIHIENSIS

(30) Priority: 12.09.2006 EP 06019071
(43) Date of publication of application: 17.06.2009
(73) Proprietor: DSM IP Assets B.V., 6411 TE Heerlen (NL)
(72) Inventor: VAN ASSEMA, Friso, Bernard, Jan, 6166 XK Geleen (NL); SEREINIG, Natascha, 6221 JE Maastricht (NL)
(74) Representative: Hoogendam, Gerrie Christine
(86) International application number: PCT/EP2007/007945
(87) International publication number: WO 2008/031578

(56) References cited:
- EP-A- 1 085 095
- WO-A-2006/069799
- DATABASE EMBL [Online] 30 May 2006 (2006-05-30), HOU, S. ET AL.: "Histidine ammonia lyase" XP002419453 Database accession no. Q5QXE5

## Description

The invention relates to a process for the preparation of an enantiomerically enriched phenylalanine compound wherein the corresponding cinnamic acid derivative is reacted with an amino group donor in the presence of a stereoselective phenylalanine ammonia lyase.

Enantiomerically enriched phenylalanine compounds are important intermediates for for example pharmaceutical products, in particular in the preparation of angiotensin I converting enzyme inhibitors (ACE), which are used for the treatment of high blood pressure (hypertension). Optically active (*S*)-indoline-2-carboxylic acid, which can be prepared by ring closure of (*S*)- 2-chlorophenylalanine, for instance, can be used in the preparation of [2*S*-[1[R*(R*), 2α, 3aβ, 7aβ]]-1-[2-[[1-(ethoxycarbonyl) butyl] amino]-1-oxopropyl] octahydro-1*H*-indole-2-carboxylic acid, also known under the tradename Perindopril, that is useful as an antihypertensive (ACE inhibitor) or cardiotonic active ingredient. Other useful applications are, for example, Pentopril and Indolapril.

A process for the preparation of an enantiomerically enriched phenylalanine compound is known from WO2006/069799 A1, wherein in a specific example (S)-2-amino-3-(2-chloro-phenyl)-propionic acid is prepared by reacting 3-(2-chloro-phenyl)acrylic acid with aqueous NH₃ in the presence of phenylalanine ammonia lyase (PAL) from *R. glutinis.* The amino acid sequence of PAL from *Rhodotorula glutinis* can be found in WO 01/11071 A2 and in SEQ ID NO. 9 herein.

A disadvantage of the process as described in WO2006/069799 A1 is that substrate inhibition occurs. Therefore, the process needs to be run at low concentration. For the production of an acceptable amount of product, large volumes or complicated feeding protocols are therefore required, making it less attractive from a commercial point of view.

Therefore, it is the object of the present invention to provide a process for the preparation of a phenylalanine compound wherein substrate inhibition is decreased.

This object has been achieved by a process for the preparation of an enantiomerically enriched phenylalanine compound by reacting the corresponding cinnamic acid with an amino group donor in the presence of a stereoselective phenylalanine ammonia lyase having a sequence identity of at least 50% with SEQ ID NO. 4.

The process of the present invention therefore provides an improved and/or commercially attractive process for the preparation of a phenylalanine compound. Substrate inhibition, that is -a decrease in initial activity of the enzyme upon an increase in substrate concentration-, is decreased by using a PAL having at least 50% identity with SEQ ID NO. 4. Furthermore, phenylalanine ammonia lyases (PALs) having at least 50% identity with SEQ ID NO. 4 are easily over-expressed in *E. coli.* Another advantage of the PALs having at least 50% identity with SEQ ID NO. 4 as compared to PAL from *R. glutinis* is that they have a higher activity.

The % identity as defined herein is determined in sequence alignment studies using

Clone Manager 6 (version 6.00/align Plus 4, version 4.10 (Scientific & Educational Software) using BLOSUM62 as scoring matrix.

Preferably, in the process of the present invention, the PAL used has a sequence identity of at least 50%, more preferably at least 53%, more preferably at least 55%, more preferably at least 57%, even more preferably at least 60%, even more preferably at least 62%, even more preferably at least 65%, even more preferably at least 70%, in particular at least 75%, more in particular at least 80%, more in particular at least 85%, more in particular at least 90%, even more in particular at least 95%, even more in particular at least 97%, even more in particular at least 98%, even more in particular at least 99%, most in particular 100% with SEQ ID NO. 4.

PAL sequences as can be used in the process of the present invention can easily be identified by the person skilled in the art, for example using BLAST searches in a database, for example the NCBI database. The nucleic acid sequences encoding for these PAL sequences can easily be identified, for example by using the programmes available via BLAST at the NCBI. Alternatively, starting from the amino acid sequence, a nucleic acid sequence can also be synthesized in the preferred or optimised codon usage for the desired host cell, for example by commercial companies such as GenScript or DNA 2.0. The nucleic acid sequence encoding the amino acid sequence of a PAL enzyme used in the process of the present invention, can be cloned in a suitable vector and after introduction in a suitable host, the sequence can be (over)expressed according to standard cloning and expression techniques, which are known to the person skilled in the art (e.g., as described in Sambrook, J., Fritsh, E. F., and Maniatis, T. Molecular Cloning: A Laboratory Manual.2nd, ed., Cold Spring Harbor Laboratory, Cold Spring Harbor Laboratory Press, Cold Spring Harbor, NY, 1989) to produce a PAL enzyme having at least 50% sequence identity with SEQ ID NO. 4.

In the framework of the invention, PAL (phenylalanine ammonia lyase) is defined as an enzyme with phenylalanine ammonia lyase activity, i.e. the ability to catalyse the reversible non-reductive amination of cinnamic acid to phenylalanine. HAL (histidine ammonia lyase) is defined as an enzyme with histidine ammonia lyase activity, i.e. the ability to catalyze the reversible non-reductive amination of urocanoic acid to histidine.

Preferably, the phenylalanine compound produced in the process of the present invention is a compound of formula (1) or a salt thereof 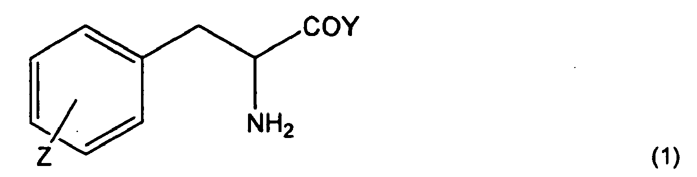 wherein Y stands for an hydroxyl group, a substituted or non-substituted alkoxy group with 1-10 carbon atoms, a substituted or non-substituted aryloxy group, or an amine group or an amine residue -NHR wherein R is an alkyl, aralkyl, aryl, akaryl group comprising 1-10 C atoms and optionally one or more heteratoms, and wherein Z represents one or more substituents on an aromatic group that are chosen from hydrogen, an hydroxyl group, an optionally substituted cyclic or acyclic aliphatic group optionally containing one or more heteroatoms, with 1-10 carbon atoms, an optionally substituted aliphatic heterocyclic group with 1-10 carbon atoms an optionally substituted (hetero)aryl group, an optionally substituted alkoxy group with 1-10 carbon atoms, a halogen atom, an amine group, a nitro group, a carboxylic acid, a carboxylic ester, a carboxylic amide, nitril or trifluoromethyl group. The optional substitution in case of Z being a cyclic or acyclic aliphatic group, an aliphatic heterocyclic group, an (hetero)aryl group or an alkoxy group and in case of Y being an alkoxy or aryloxy group may for example be chosen from a trihalogenide, for example trifluoromethyl, trichloromethyl; a halogen atom, for example fluoride, chloride or iodide; an amine group, for example methylamine, dimethylamine, benzylamine, ethylamine, propylamine, phenylamine, 4-fluorophenylamine; an alkoxy or aryloxy group, for example, methoxy, ethoxy, isopropoxy, benzyloxy, phenoxy, 4-fluorophenoxy, 3-fluorophenoxy, 2-fluorophenoxy; a thioether, for example methyl thioether, ethyl thioether, benzyl thioether; an trihaloalkylether, for example trifluoromethylether; an aryl or heteroaryl, for example, phenyl, 4-fluorophenyl, 3-fluorophenyl, 2-fluorophenyl, 2-trifluoromethylphenyl, 3-trifluoromethylphenyl, 4-trifluoromethylphenyl, thiophene, furan, pyrazole, pyrole, imidazole, or the like. Preferred are compounds in which Z is hydrogen, a halogen atom, trifluoromethyl, hydroxy, methoxy or nitril and Y is hydroxyl.

More preferably, the phenylalanine compound produced in the process of the present invention is a compound of formula (2) or a salt thereof: 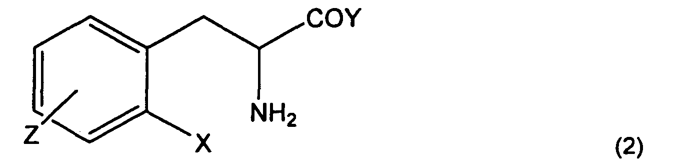 wherein Y is as defined above for formula (1) and wherein Z is as defined above for formula (1) and wherein X stands for a leaving group, for instance a leaving group chosen from the group of fluoride, bromide, chloride or iodine, a mesylate group, a nosylate group, a tosylate group, a triflate group, boronic acid, a nitro group and the like.

The term "leaving group" in the context of this invention means a group suitable to act as a leaving group in a process for the preparation of an enantiomerically enriched optionally substituted indoline-2-carboxylic acid according to formula (3) 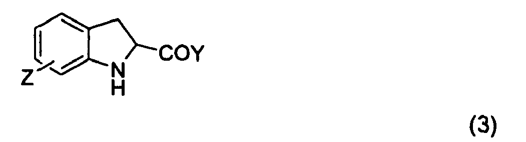 or a salt thereof, in which Y and Z are as defined above
wherein an enantiomerically enriched chiral ortho-X-substituted phenylalanine compound according to formula (2) is subjected to cyclisation at a temperature of below about 140°C, upon formation of the enantiomerically enriched indoline-2-carboxylic acid compound according to formula (3). Any substituents that may be present in Y or Z in formula (3) are as defined below. This cyclisation reaction has been disclosed in WO 2006/069799 A1.

Suitable examples of salts of compounds according to formulae (1), (2) and (3) are for example, the HCl salt, HBr salt, the ammonium salt, acetic acid salt, methane sulphonic acid salt, benzene sulphonic acid salt, the p-toluenesulphonic acid salt, maleic acid salt, tartaric acid salt, citric acid salt, or gluconic acid salt thereof.

Where Z in formula (1), (2) or (3) is a substituted cyclic or acyclic aliphatic group with 1-10 carbon atoms, a substituted aliphatic heterocyclic group with 1-10 carbon atoms, a substituted (hetero)aryl group, a substituted alkoxy group with 1-10 carbon atoms, that contains one or more heteroatoms, these heteroatoms will each independently typically be chosen from the group of P, N, O and S. Where Y contains one or more hetero atoms, these heteroatoms will also each independently typically be chosen from the group of P, N, O and S

Preferably, in the above formula(e), Y stands for OH or OR¹, wherein R' stands for an alkyl, typically with 1-10 C-atoms, preferably an alkyl with 1-6 C-atoms, most preferably Y stands for OH. Preferably, Z stands for H or halogen, for example fluoride, chloride, bromide or iodide, most preferably Z stands for H. Preferably, X in formula (2) stands for fluoride, bromide, chloride or iodine, most preferably for chloride or bromide. Because of its low cost, and/or from an environmental point of view, chloride is most preferred.

Suitable examples of phenylalanine compounds are for example 2-bromo-phenylalanine, 2-chlorophenylalanine, 2-iodo-phenylalanine, 2-bromophenylalanine methyl ester, 2-chlorophenylalanine methyl ester, 2-iodo-phenylalanine methyl ester, 2-bromophenylalanine ethyl ester, 2-chlorophenylalanine ethyl ester, 2-iodo-phenylalanine ethyl ester, 2-bromophenylalanine benzyl ester, 2-chlorophenylalanine benzyl ester, 2-iodo-phenylalanine benzyl ester, 2-bromophenylalanine amide, 2-chlorophenylalanine amide, 2-iodo-phenylalanine amide, or the like. Preferred are 2-bromophenylalanine, 2-chlorophenylalanine, 2-bromophenylalanine benzyl ester and 2-chlorophenylalanine benzyl ester. More preferred are 2-bromophenylalanine and 2-chlorophenylalanine. Even more preferred are the (S)-enantiomers of the respective examples of phenylalanine compounds. Most preferred are (S)-2-chlorophenylalanine and (S)-2-bromophenylalanine.

"enantiomerically enriched" as defined herein is equivalent to the term "optically active" and means that one of the enantiomers of a compound is present in excess compared to the other enantiomer. This excess will hereinafter be referred to as "enantiomeric excess" or e.e. (as for example determined by chiral GLC or HPLC analysis). The enantiomeric excess e.e. is equal to the difference between the amounts of enantiomers divided by the sum of the amounts of the enantiomers, which quotient can be expressed as a percentage after multiplication by 100.

In the process of the invention, the optical purity of the phenylalanine compound is preferably at least about 50%, more preferably at least about 75%, even more preferably at least about 90%, in particular at least about 95 %, most in particular at least about 98%.

Suitable examples of salts of a phenylalanine compound of formula (1) or (2) are for example, the HCl salt, HBr salt, acetic acid salt, methane sulphonic acid salt, benzene sulphonic acid salt, the p-toluenesulphonic acid salt, maleic acid salt, tartaric acid salt, citric acid salt, or gluconic acid salt thereof.

Suitable amino group donors include for example NH₃ or NH₄OH or B-NH₂, wherein B is a -COR¹ group in which R¹ may be chosen from an optionally substituted cyclic or acyclic aliphatic group with 1-10 carbon atoms, an optionally substituted aliphatic heterocyclic group with 1-10 carbon atoms, an optionally substituted (hetero)aryl group, an optionally substituted alkoxy group with 1-10 carbon atoms, an optionally substituted sulfonyl group or the like. Preferred are NH₃ and NH₄OH. It is of course also possible to use a combination of various amino group donors. The optionally present substituents on R¹ are may be chosen from an hydroxyl group, a trihalogenide, for example trifluoromethyl, trichloromethyl; a halogen atom, for example fluoride, chloride or iodide; an amine group, for example methylamine, dimethylamine, benzylamine, ethylamine, propylamine, phenylamine, 4-fluorophenylamine; an alkoxy or aryloxy group, for example, methoxy, ethoxy, isopropoxy, benzyloxy, phenoxy, 4-fluorophenoxy, 3-fluorophenoxy, 2-fluorophenoxy; a thioether, for example methyl thioether, ethyl thioether, benzyl thioether; an trihaloalkylether, for example trifluoromethylether; an aryl or heteroaryl, for example, phenyl, 4-fluorophenyl, 3-fluorophenyl, 2-fluorophenyl, 2-trifluoromethylphenyl, 3-trifluoromethylphenyl, 4-trifluoromethylphenyl, thiophene, furan, pyrazole, pyrole, imidazole, or the like.

With 'stereoselectivity' of PAL is meant that PAL preferentially catalyses the formation of one of the enantiomers of the compound (2). The stereoselectivity of an enzyme may be expressed in terms of E-ratio, the ratio of the specificity constants Vₘₐₓ /Kₘ of the two enantiomers as described in C-S. Chen, Y Fujimoto, G. Girdaukas, C. J. Sih., J. Am. Chem. Soc. 1982, 104, 7294-7299. In the framework of the invention, the E-ratio of a PAL is determined using the Vₘₐₓ and Kₘ values as determined in the non-oxidative deamination of the ortho-X-substituted phenylalanine compound (= compound (1)) using PAL.

Preferably, PAL forms products with an e.e. of > 80%, more preferably , PAL forms products with an e.e. of >90%, even more preferably, PAL forms products with an e.e. of >95%, most preferably, PAL forms products with an e.e. of >99% Preferably, PAL stereoselectively catalyses the formation of the (S)-enantiomer of the phenylalanine compound, more preferably, ortho-chloro-substituted (*S*)-phenylalanine compound is formed.

The PAL as used in the process of the present invention preferably does not suffer from substrate inhibition, i.e. preferably does not suffer from inhibition by cinnamic acid or derivatives thereof.

The process of the invention is in no way limited by the form in which the PAL having at least 50% identity with SEQ ID NO. 4 is used. The PAL may for example be present in a form chosen from a crude enzyme solution, a purified enzyme solution, (permeabilized and/or immobilized) cells that naturally or through genetic modification possess PAL activity, in a lysate of cells with such activity, in immobilized form, in chemically modified form or further forms known to a person skilled in the art, or in a combination of two or more of these forms.

It will be clear to the person skilled in the art that use can also be made of mutants of naturally occurring (wild type) PAL enzymes. For example, it is possible to make PAL-activity mutants, i.e. mutants which show a higher activity than wild type PAL on at least one substrate, PAL-expression mutants, i.e. mutants which are better expressed than wild type PAL; and the like. PAL-activity-mutants of wild-type enzymes can for example be made by modifying the DNA encoding the wild type enzymes using mutagenesis techniques known to the person skilled in the art (random mutagenesis, site-directed mutagenesis, directed evolution, gene shuffling, etc.) so that the DNA encodes an enzyme that differs by at least one amino acid from the wild type enzyme and by expressing the thus modified DNA in a suitable (host) cell.

Alternatively, PAL-expression mutants may be prepared by modifying the DNA encoding wild type PAL enzymes using mutagenesis techniques known to the person skiled in the art, such that if the DNA is expressed in a (host) cell, the level of expression of PAL is higher for the mutated DNA than for the non-mutated DNA. The skilled person knows how to select these PAL-expression mutants, for example by measuring the relative activity for at least three different substrates and by comparing the ratio of the activities with the wild type enzyme. Expression mutants have a higher activity for all the different substrates, while the ratio of the activities between the substrates is equal to that of the wild type enzyme. Mutants of the PAL enzyme may have improved properties with respect to (stereo)selectivity and/or activity and/or stability and/or solvent resistance and/or pH profile and/or temperature profile, and/or expression and/or product and/or substrate inhibition and said mutants of PAL may be specifically selected based on the presence of any of these improved properties alone or in combination of two or more thereof.

It may be of advantage to add a reducing agent to the process of the present invention in order to reduce negative effects of oxygen on the PAL life. Examples of such reducing agents include hydrogen sulfide, thioglycolic acid, thiosulfuric acid, nitrous acid, sulfurous acid, ammonium and metal salts of the above, dithiothreitol, ethylmercaptan, ethylenemercaptan, methylmercaptan, 2-mercaptoethanol, hydrogen, nitrous oxide, iron (II) compound, manganese (II) compounds, sulfur and zinc. Preferably, to reduce the effects of oxygen on the PAL life, the process of the present invention may be performed under anaerobic conditions in a manner known to the person skilled under the art, for example, by performing the process of the present invention under a nitrogen atmosphere. The person skilled in the art can perform routine experiments to choose the optimal reaction conditions with respect to- amongst others- temperature, pH, concentration, and solvents.

Preferably, the temperature of the PAL-step is chosen such that the activity of the chosen PAL is optimal. For example, the preferred reaction temperature for the PAL of *Idiomarina loihiensis* lies in the range from 0 to 50°C, in particular from 25 to 45°C, most in particular from 27 to 37 °C.

Preferably, the pH of the PAL step and the concentration of the amino group donor (preferably NH₃) are chosen as such that the activity of the chosen PAL and the stability of formed phenylalanine compound and the yields in which these compounds are obtained are optimal. For example, the pH for the PAL of *Idiomarina loihiensis* lies preferably in the range from pH 8 to pH 14, in particular in the range from pH 10 to 12, more in particular in the range from pH 10.5 to 11.5.

For example, the ammonium hydroxide (NH₄OH) concentration in water for the PAL of *Idiomarina loihiensis* is preferably chosen between about 5 and about 25 wt. %, in particular from about 10 to 15 wt. %, most in particular from about 12 to 14 wt.%.

The process of the present invention may for example be performed in aqueous ammonia, to which optionally a co-solvent, a solubilization agent or an additive may be added in order to enhance to solubilization of the corresponding cinnamic acid compound or to enhance the PAL activity or stability. Examples of cosolvents include dimethylformamide, tetrahydrofuranacetonitrile, glutaraldehyde, glycerol, toluene, ethanol, acetone, ether and dimethylsulfoxide (DMSO). Examples of solubilization agents include Triton X100 (made by Aldrich Chemical Company, Inc., Milwaukee, Wis), Tween-80 (made by Aldrich Chemical Company, Inc., Milwaukee, Wis), polyethylene glycol (PEG). Examples of additives include glutamic acid, isoleucine, ethylene glycol and sorbitol.

The phenylalanine compound as obtained in the process of the present invention may be used directly in a next step or may be purified first. The phenylalanine compound may be purified from the reaction mixture obtained in the process of the present invention using conventional methods known to the person skilled in the art. For example, the reaction mixture obtained comprising the phenylalanine compound may be filtered or centrifuged (to remove the undissolved particles, such as for instance the host cells expressing PAL). Further, the amino group donor may for example be evaporated after which the phenylalanine compound may be isolated, preferably by precipitation.

The yield obtained with the process of the present invention is preferably at least 30%, more preferred at least 40%, even more preferred at least 50%, in particular preferred at least 60%, more in particular preferred at least 80%, most in particular at least 90%.

In a preferred embodiment of the process of the present invention ortho-chloro-substituted (*S*)-phenylalanine compound (formula (2) wherein X stands for Cl and Z stands for H) is prepared by reacting the corresponding 2-chloro-substituted cinnamic acid compound in an aqueous solution of ammonium hydroxide, the ammonium hydroxide concentration in water being from about 12 to 14 wt.% in the presence of PAL from *Shewanella oneidensis, Idiomarina loihiensis, Fibrobacter succinogenes* or *Vibrio vulnificus,* the amino acid sequences of which are given in respectively SEQ ID NO. 2 (having a sequence identity of 60 % with SEQ ID NO. 4), SEQ ID NO. 4, SEQ ID NO. 6 (having a sequence identity of 53 % with SEQ ID NO. 4), and SEQ ID NO. 8 (having a sequence identity of 63 % with SEQ ID NO. 4). Surprisingly, although these four enzymes were annotated as histidine ammonia lyases in the GenBank database from NCBI, these four enzymes have phenylalanine ammonia lyase activity. Preferably in this embodiment, the temperature is chosen between 25 to 45°C and the pH ranges from pH 10.5 to 11.5.

The disclosure also relates to a process for the preparation of an enantiomerically enriched phenylalanine compound according to the invention in the presence of a stereoselective phenylalanine ammonia lyase having a sequence identity of at last 80%, preferably 90%, most preferably 95% with SEQ ID NO. 2, SEQ ID NO.4, SEQ ID NO. 6 or SEQ ID NO. 8.

As disclosed in WO 2006/069799 A1, the phenylalanine compound formed in the process of the present invention can be used to prepare optically active (*S*)-indoline-2-carboxylic acid, which on it's turn is an intermediate in the production of [2*S*-[1[R*(R*), 2α, 3aβ, 7aβ]]-1-[2-[[1-(ethoxycarbonyl) butyl] amino]-1-oxopropyl] octahydro-1H-indole-2-carboxylic acid.
Therefore, the invention also relates to a process comprising the process of the present invention, wherein the phenylalanine compound produced is further converted into an active pharmaceutical ingredient, in particular [2*S*-[1[R*(R*), 2α, 3aβ, 7aβ]]-1-[2-[[1-(ethoxycarbonyl) butyl] amino]-1-oxopropyl] octahydro-1*H*-indole-2-carboxylic acid.

### Examples

The following clones were used in tests for PAL activity.

| **Clone nr** | **Enzyme** / **source organism** | **SEQ ID NO Amino acid sequence** | **SEQ ID NO Nucleic acid sequence** |
|---|---|---|---|
| 1 | *Shewanella oneidensis* | 2 | 1 |
| 2 | *Idiomarina loihiensis* | 4 | 3 |
| 3 | *Fibrobacter succinogenes* | 6 | 5 |
| 4 | *Vibrio vulnificus* | 8 | 7 |
| 5, comparative example | *Rhodotorula glutinis* in pUC57 | 10 (see also WO01/11071A2) | 9 |

The genes having the nucleic acid sequences as given above were amplified by PCR and the purified PCR products were fused with the *Nco*I and *Bgl*II sites of vector pSE420 (Invitrogen Corp. CA USA), using the Xi cloning method. By using this method, the *Ncol* site is destroyed after cloning of the genes. The formed plasmids were transformed to *E. coli* TOP 10 cells (Invitrogen), and checked for the right insert by restriction analysis and agarose gel electrophoresis to give the clones with the clone number as given above.

The PAL gene of *R. glutinis* was synthesized by Genscript Corporation, Scotch Plains, USA according to the protein sequence of the PAL of *Rhodosporidium toruloides* ATCC 10788 which sequence is listed in the protein database of the NCBI under number CAA31209. The codons of the synthetic PAL gene were optimised for expression in *E. coli.* The synthesized gene was ligated in the Smal site of pUC57 by Genscript to give the pUC57_PAL construct. The lyophilized plasmid (5 µg) (obtained from Genscript) was dissolved in 50 µl MilliQ water and 1 µl of this solution was used to transform an aliquot of *E. coli* DH5α chemicallly competent cells (Invitrogen, Breda, The Netherlands) according to the protocol of the supplier.

### Preparation of cells and cell free extract

### Cultivation of E. coli containing the PALs

*E. coli* containing the corresponding plasmids were inoculated each in 20m12xTY medium (16g/l Tryptone; 10g/l yeast extract, 7g/l NaCl) supplemented with carbeniciline (100µg/ml) and incubated overnight at 28°C. These cultures were used to inoculate larger volumes of medium (250µl pre-culture for 250ml medium). The cells were grown at 37°C until the OD reached ~1.). The PAL clones of *Shewanella oneidensis* (clone nr 1), *Idiomarina loihiensis* (clone nr 2), *Fibrobacter succinogenes* (clone nr 3), *Vibrio vulnificus* (clone nr 4) were induced with IPTG (0.5mM end concentration). The PAL clone of *Rhodotorula glutinis* was induced with IPTG (1mM end concentration). After induction the cultures were shaken at 28°C over night.

The cells (~2g / 250ml medium) were harvested by centrifugation (8000 RPM at 4°C), and re-suspended in 0,1 M Tris / HCl buffer pH 8,8 (1 ml buffer /0.5g cells). The cell suspentions were divided in aliquots of 0,2 ml and centrifuged. The supernatant was discarded, and the cell pellet (about 0,1 gram each) was stored at -20 °C.

### Protein determination, SDS Page, preparation of CFE

0,1g cells were resuspended in 1 ml 0,1 M Tris / HCl buffer pH 8,8 and the cells were disrupted by sonification. A part of the sonificated cells was centrifuged and the Cell Free Extract (CFE) was decanted. The protein concentration in the different fractions was determined by the method described by Bradford.

To determine the level of expression, 25 µg of protein of the Cell Free Extracts and the total cell extracts were applied to a NuPAGE gel.

| Strain | nr | expression |
|---|---|---|
| *Shewanella oneidensis* | 1 | ++ |
| *Idiomarina loihiensis* | 2 | ++++ |
| *Fibrobacter succinogenes* | 3 | +++ |
| *Vibrio vulnificus* | 4 | +++ |
| *Rhodotorula glutinis* | 5 | ++ |

| | | |
|---|---|---|
| ++++ > 50% of total protein; +++ = 30-50% of total protein; ++ = 10-30% of total protein; | | |

This analysis shows that the PAL genes are well expressed in *E. coli.* No inclusion bodies were formed. Clone 2 *(Idiomarina loihiensis)* showed the highest expression levels in *E. coli.*

### Initial activities at pH 9.25

A reaction solution was prepared by adjusting a 10 wt% ammonia solution to pH 9.25 with sulphuric acid at 30°C.

10mM trans-2-bromo-cinnamic acid solutions were prepared in the ammonia solution. 4,8ml of the substrate solution was warmed to 30°C. 100mg of a cell pellet was suspended in 100µl corresponding ammonia solution and added to the reaction mixture. The reaction was followed in time. The formed (*L*)-2-bromophenylalanine was determined by HPLC analysis.

The relative initial activities (compared to the activity of *E. coli* cells containing *R. glutinis* PAL) are measured over the first 30-60 minutes of the reaction.

**Table 1: Relative initial activities of individual PAL clones compared to the PAL of R. glutinis.**

| PAL clone | Rel initial act (%) |
|---|---|
| 4. *S. oneidensis* | 10900 |
| *12. I. loihiensis* | 14300 |
| *16. F.succinogenes* | 11400 |
| *18. V. vulnificus* | 8400 |
| 19. *R. glutinis* | 100 |

As can be seen from Table 1, the genes cloned from S. *oneidensis, I. loihiensis, F. succinogenes* and V. *vulnificus* were all much more active than the gene cloned from *R*. *glutinis* under the conditions tested.

### Determination of substrate inhibition

For reactions at pH 11: 4,8ml of 100mM, 50mM, 20mM and 10mM solutions of trans-2-bromo-cinnamic acid in 13 wt% ammonia (adjusted to pH 11 with sulphuric acid at 30°C) were warmed to 30°C.

For reactions at pH 9.25: Solid trans-2-bromo-cinnamic acid was suspended in 4,8ml 8 wt% ammonia (adjusted to pH 9,25 with sulphuric acid at 30°C) to give a final concentrations of 50mM, 20mM and 10mM. The reaction mixtures were warmed to 30°C. To start the reaction a suspension of 100mg cell pellet in 100µl corresponding ammonia solution was added. The reaction was followed in time. The formed (*L*)-2-bromo-phenylalanine was determined by HPLC analysis.

Initial activities (relative to the PAL clone of *R. glutinis)* in 13% NH3 pH11 at different substrate concentrations:

| Clone | 10 mM | 20 mM | 50 mM | 100 mM |
|---|---|---|---|---|
| *I. loihiensis* | 104 | 197 | 423 | 594 |
| *R. glutinis* | 100 | 38 | 8 | 0 |

Initial activities (relative to the PAL clone of *R. glutinis)* in 8% NH3 pH9,27 at different substrate concentrations:

| Clone | 10 mM | 20 mM | 50 mM |
|---|---|---|---|
| *S. oneidensis* | 8350 | 12460 | 11650 |
| *I. loihiensis* | 14300 | 20000 | 24110 |
| *F. succinogenes* | 7340 | 12380 | 13530 |
| *V. vulnificus* | 6820 | 8770 | 8860 |
| *R. glutinis* | 100 | nd* | nd |

| | | | |
|---|---|---|---|
| * nd: The activity was not determined due to the very low activity under these conditions NOTE: Reactions with 8% ammonia pH 9.27 were slurries above concentrations of 20mM. | | | |

As can be seen from the above results, all PALs showed less substrate inhibition than *R. glutinis* PAL. The PAL cloned from *I. loihiensis* shows an exceptional tolerance to high substrate concentrations even at high pH values, where the solubility of the substrate is high.

## Claims

1. Process for the preparation of an enantiomerically enriched phenylalanine compound by reacting the corresponding cinnamic acid with an amino group donor in the presence of a stereoselective phenylalanine ammonia lyase having a sequence identity of at least 50% with SEQ ID NO. 4.

2. Process according to claim 1, wherein the phenylalanine compound is a compound of formula (1) or a salt thereof 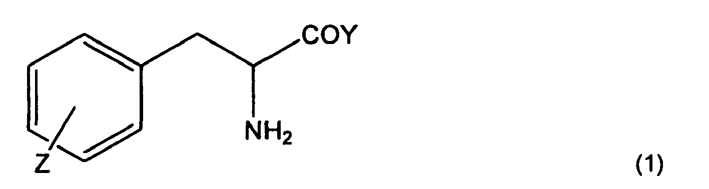 wherein Y stands for an hydroxyl group, a substituted or non-substituted alkoxy group with 1-10 carbon atoms, a substituted or non-substituted aryloxy group, or an amine residue and wherein Z represents one or more substituents on an aromatic group that are chosen from hydrogen, an hydroxyl group, an optionally substituted cyclic or acyclic aliphatic group with 1-10 carbon atoms, an optionally substituted aliphatic heterocyclic group with 1-10 carbon atoms, an optionally substituted (hetero)aryl group, an optionally substituted alkoxy group with 1-10 carbon atoms, a halogen atom, an amine group, a nitro group, a carboxylic acid, a carboxylic ester, a carboxylic amide, nitril or trifluoromethyl group.

3. Process according to claim 1, wherein the phenylalanine compound is a compound of formula (2) or a salt thereof 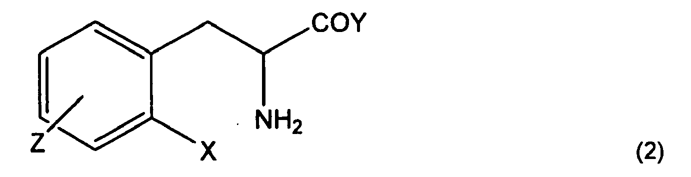 wherein Z stands for one or more substituents on an aromatic group that are chosen from hydrogen, an hydroxyl group, an optionally substituted cyclic or acyclic aliphatic group with 1-10 carbon atoms, an optionally substituted aliphatic heterocyclic group with 1-10 carbon atoms, an optionally substituted (hetero)aryl group, an optionally substituted alkoxy group with 1-10 carbon atoms, a halogen atom, an amine group, a nitro group, a carboxylic acid, a carboxylic ester, a carboxylic amide, nitril or trifluoromethyl group and wherein Y stands for an hydroxyl group, a substituted or non-substituted alkoxy group with 1-10 carbon atoms, a substituted or non-substituted aryloxy group, or an amine residue, and wherein X stands for a leaving group, for instance a leaving group chosen from the group of halogen, mesylate, nosylate, tosylate, triflate, boronic acid, a nitro group and the like.

4. Process according to claim 3, wherein X stands for chloride or bromide, Y stands for hydroxyl and Z stands for hydrogen.

5. Process according to claim 4, wherein the compound of formula (1) is (*S*)-2-chlorophenylalanine or (*S*)-2-bromophenylalanine.

6. Process according to any one of claims 1-5, wherein the amino group donor is NH₃ or NH₄OH.

7. Process according to any one of claims 1-6, wherein the stereoselective phenylalanine ammonia lyase has the amino acid sequence as presented in SEQ ID NO. 2, SEQ ID NO. 4, SEQ ID NO. 6 or SEQ ID NO. 8.

8. Process according to any one of claims 3-7, further comprising the step of, subjecting to cyclisation an enantiomerically enriched chiral ortho-X-substituted phenylalanine compound according to formula (2) 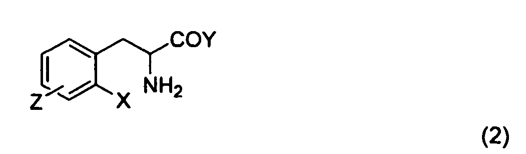 or a salt thereof, in which Y and Z are as defined above and X is a leaving group, at a temperature of below about 140°C, whereby an enantiomerically enriched indoline-2-carboxylic acid compound according to formula (3) 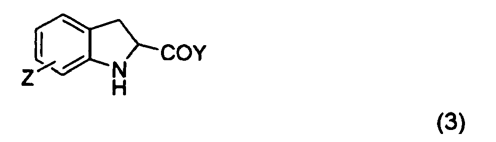 is formed.

9. Process for the production of an active pharmaceutical ingredient, comprising the process of any one of claims 1-8.

## Patentansprüche

1. Verfahren zur Herstellung einer enantiomerenangereicherten Phenylalaninverbindung, bei dem man die entsprechende Zimtsäure in Gegenwart einer stereoselektiven Phenylalanin-Ammoniak-Lyase mit einer Sequenzidentität von wenigstens 50% mit SEQ ID Nr. 4 mit einem Aminogruppendonor umsetzt.

2. Verfahren nach Anspruch 1, wobei es sich bei der Phenylalaninverbindung um eine Verbindung der Formel (1) oder ein Salz davon handelt, 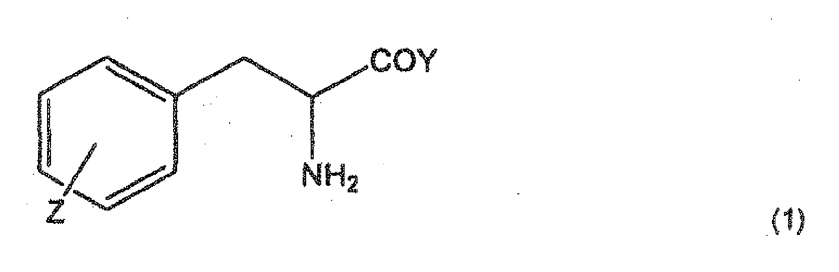 wobei Y für eine Hydroxylgruppe, eine substituierte oder nicht substituierte Alkoxygruppe mit 1-10 Kohlenstoffatomen, eine substituierte oder nicht substituierte Aryloxygruppe oder einen Aminrest steht und wobei Z für einen oder mehrere Substituenten an einer aromatischen Gruppe steht, die ausgewählt sind aus Wasserstoff, einer Hydroxylgruppe, einer gegebenenfalls substituierten cyclischen oder acyclischen aliphatischen Gruppe mit 1-10 Kohlenstoffatomen, einer gegebenenfalls substituierten aliphatischen heterocyclischen Gruppe mit 1-10 Kohlenstoffatomen, einer gegebenenfalls substituierten (Hetero)arylgruppe, einer gegebenenfalls substituierten Alkoxygruppe mit 1-10 Kohlenstoffatomen, einem Halogenatom, einer Amingruppe, einer Nitrogruppe, einer Carbonsäure, einem Carbonsäureester, einem Carboxamid, einem Nitril oder einer Trifluormethylgruppe.

3. Verfahren nach Anspruch 1, wobei es sich bei der Phenylalaninverbindung um eine Verbindung der Formel (II) oder ein Salz davon handelt, 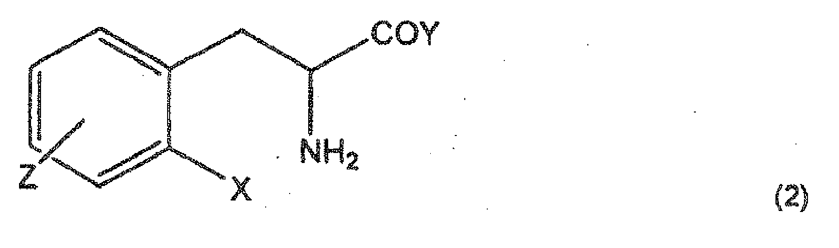 wobei Z für einen oder mehrere Substituenten an einer aromatischen Gruppe steht, die ausgewählt sind aus Wasserstoff, einer Hydroxylgruppe, einer gegebenenfalls substituierten cyclischen oder acyclischen aliphatischen Gruppe mit 1-10 Kohlenstoffatomen, einer gegebenenfalls substituierten aliphatischen heterocyclischen Gruppe mit 1-10 Kohlenstoffatomen, einer gegebenenfalls substituierten (Hetero)arylgruppe, einer gegebenenfalls substituierten Alkoxygruppe mit 1-10 Kohlenstoffatomen, einem Halogenatom, einer Amingruppe, einer Nitrogruppe, einer Carbonsäure, einem Carbonsäureester, einem Carboxamid, einem Nitril oder einer Trifluormethylgruppe und wobei Y für eine Hydroxylgruppe, eine substituierte oder nicht substituierte Alkoxygruppe mit 1-10 Kohlenstoffatomen, eine substituierte oder nicht substituierte Aryloxygruppe oder einen Aminrest steht und wobei X für eine Abgangsgruppe, zum Beispiel eine Abgangsgruppe ausgewählt aus der Gruppe Halogen, Mesylat, Nosylat, Tosylat, Triflat, Boronsäure, eine Nitrogruppe und dergleichen, steht.

4. Verfahren nach Anspruch 3, wobei X für Chlorid oder Bromid steht, Y für Hydroxyl steht und Z für Wasserstoff steht.

5. Verfahren nach Anspruch 4, wobei es sich bei der Verbindung der Formel (1) um (*S*)-2-Chlorphenylalanin oder (*S*)-2-Bromphenylalanin handelt.

6. Verfahren nach einem der Ansprüche 1-5, wobei es sich bei dem Aminogruppendonor um NH₃ oder NH₄OH handelt.

7. Verfahren nach einem der Ansprüche 1-6, wobei die stereoselektive Phenylalanin-Ammoniak-Lyase die in SEQ ID Nr. 2, SEQ ID Nr. 4, SEQ ID Nr. 6 oder SEQ ID Nr. 8 wiedergegebene Aminosäuresequenz aufweist.

8. Verfahren nach einem der Ansprüche 3-7, welches weiterhin den Schritt der Cyclisierung einer enantiomerenangereicherten chiralen ortho-X-substituierten Phenylalaninverbindung der Formel (2) 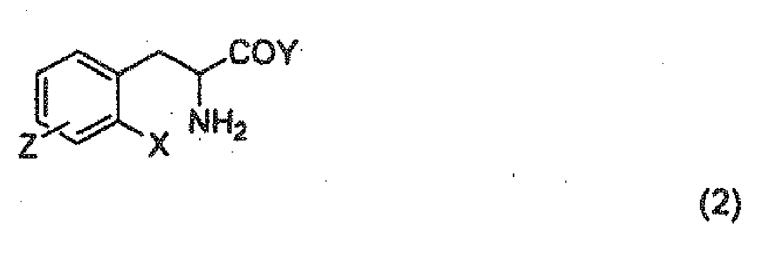 oder eines Salzes davon, in welcher Y und Z wie oben definiert sind und X für eine Abgangsgruppe steht, bei einer Temperatur von unter etwa 140°C umfasst, wobei eine enantiomerenangereicherte Indolin-2-carbonsäureverbindung der Formel (3) 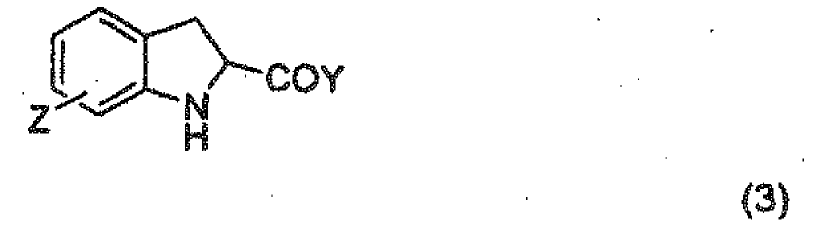 gebildet wird.

9. Verfahren zur Herstellung eines pharmazeutischen Wirkstoffs, welches das Verfahren nach einem der Ansprüche 1-8 umfasst.

## Revendications

1. Procédé pour la préparation d'un composé de phénylalanine énantiomériquement enrichi par réaction de l'acide cinnamique correspondant avec un donneur de groupe amino en présence d'une phénylalanine ammoniac lyase stéréosélective ayant une identité de séquence d'au moins 50 % avec SEQ ID n° 4.

2. Procédé selon la revendication 1, dans lequel le composé de phénylalanine est un composé de formule (1) ou un sel de celui-ci 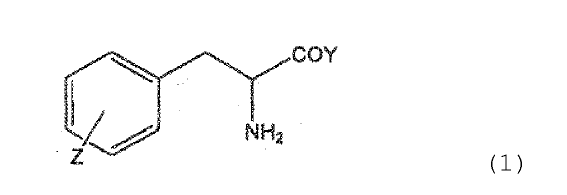 dans laquelle Y représente un groupe hydroxyle, un groupe alcoxy substitué ou non substitué ayant 1-10 atomes de carbone, un groupe aryloxy substitué ou non substitué ou un résidu d'amine et dans laquelle Z représente un ou plusieurs substituants sur un groupe aromatique qui sont choisis parmi l'hydrogène, un groupe hydroxyle, un groupe aliphatique cyclique ou acyclique facultativement substitué ayant 1-10 atomes de carbone, un groupe hétérocyclique aliphatique facultativement substitué ayant 1-10 atomes de carbone, un groupe (hétéro)aryle facultativement substitué, un groupe alcoxy facultativement substitué ayant 1-10 atomes de carbone, un atome d'halogène, un groupe amine, un groupe nitro, un acide carboxylique, un ester carboxylique, un amide carboxylique, un nitrile ou un groupe trifluorométhyle.

3. Procédé selon la revendication 1, dans lequel le composé de phénylalanine est un composé de formule (2) ou un sel de celui-ci 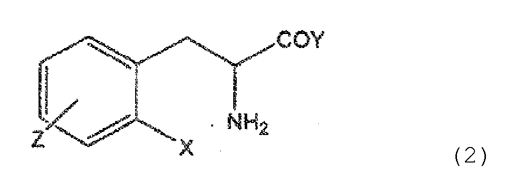 dans laquelle Z représente un ou plusieurs substituants sur un groupe aromatique qui sont choisis parmi l'hydrogène, un groupe hydroxyle, un groupe aliphatique cyclique ou acyclique facultativement substitué ayant 1-10 atomes de carbone, un groupe hétérocyclique aliphatique facultativement substitué ayant 1-10 atomes de carbone, un groupe (hétéro)aryle facultativement substitué, un groupe alcoxy facultativement substitué ayant 1-10 atomes de carbone, un atome d'halogène, un groupe amine, un groupe nitro, un acide carboxylique, un ester carboxylique, un amide carboxylique, un nitrile ou un groupe trifluorométhyle et dans laquelle Y représente un groupe hydroxyle, un groupe alcoxy substitué ou non substitué ayant 1-10 atomes de carbone, un groupe aryloxy substitué ou non substitué ou un résidu d'amine et dans laquelle X représente un groupe partant, par exemple un groupe partant choisi dans le groupe constitué par un halogène, mésylate, nosylate, tosylate, triflate, l'acide boronique, un groupe nitro et similaire.

4. Procédé selon la revendication 3, dans lequel X représente un chlorure ou un bromure, Y représente un hydroxyle et Z représente un hydrogène.

5. Procédé selon la revendication 4, dans lequel le composé de formule (1) est la (*S*)-2-chlorophénylalanine ou la (*S*) -2-bromophénylalanine .

6. Procédé selon l'une quelconque des revendications 1-5, dans lequel le donneur de groupe amino est NH₃ ou NH₄OH .

7. Procédé selon l'une quelconque des revendications 1-6, dans lequel la phénylalanine ammoniac lyase stéréosélective a la séquence d'acides aminés telle que présentée dans SEQ ID n° 2, SEQ ID n° 4, SEQ ID n° 6 ou SEQ ID n° 8.

8. Procédé selon l'une quelconque des revendications 3-7, comprenant en outre l'étape consistant à soumettre à une cyclisation un composé de phénylalanine substitué par X en position ortho chiral énantiomériquement enrichi selon la formule (2) 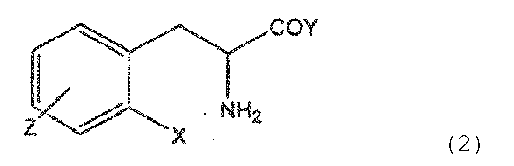 ou un sel de celui-ci, dans laquelle Y et Z sont tels que définis ci-dessus et X est un groupe partant, à une température au-dessous d'environ 140 °C, ce par quoi un composé acide indoline-2-carboxylique énantiomériquement enrichi selon la formule (3) 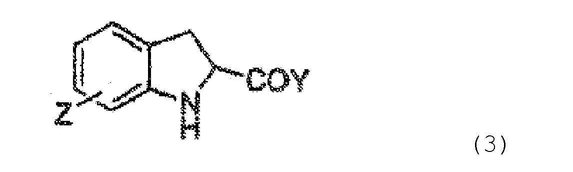 est formé.

9. Procédé pour la production d'un ingrédient pharmaceutique actif, comprenant le procédé de l'une quelconque des revendications 1-8.


### SEQUENCE LISTING

<110> DSM IP Assets BV
<120> PROCESS FOR THE PREPARATION OF A PHENYLALANINE COMPOUND
<130> 25385WO
<150> EP06019071.7 <151> 2006-09-12
<160> 10
<170> PatentIn version 3.3
<210> 1
   <211> 1566
   <212> DNA
   <213> Shewanella oneidensis
<400> 1
   atgagccacg tagttactca aacgacaacc gttgagtcgc caatcgaatt tggccgtcag 60
   ttactcacat tagagcaggt cgtcgccgtg gctaagggcg caaaggtcaa actctgtgat 120
   gatgccgatt atcaagaata tatccaaaag ggcgcccgct ttatcgatag tttgctgcac 180
   gaagaaggcg tggtctacgg cgtcaccaca ggctatggcg actcttgcac agtgaatgtg 240
   agtcttgact tggtccacga gttgccgctg cacttatccc gttttcatgg ttgtggcctt 300
   ggtgaagtct taagcgtaat gcaagcgcgc gccgtgatgg cttgccgttt aaactccctc 360
   gccattggca aatccggcgt gacatatgag ctattaaagc gcatccaaac cttgcttaat 420
   ctcaatatcg tgccagtgat ccccgaagaa ggctcagtcg gtgccagcgg agacttaacg 480
   ccactgtctt accttgccgc cgtgctggtt ggtgagcgtg aggtgattta ccaaggcgag 540
   cgccgagcca ccaaagaggt ttatcacgag ctgaatatca cgccccatgt gctacgtccc 600
   aaggaaggtt tagccctgat gaacggcacg gcagtgatga cagcgttagc ctgtttagcc 660
   tttgatcgcg cacaatattt agcgcgttta gccagccgca ttaccgccat ggcgtcgtta 720
   acccttaaag gcaactccaa ccatttcgac gatattctgt ttgccgccaa acctcatcca 780
   gggcaaaacc aaatcgccac ttggatacgg gaagacttga accaccatgt tcacccgcgc 840
   aattccgaca gattgcagga cagatattcc atccgctgtg cgccgcatat tattggcgtg 900
   ctgcaggatg cgctgccctt tatgcgccaa tttatcgaaa ccgaagttaa cagcgccaac 960
   gacaacccga ttgtcgatgc tgaaggcgag catattctcc atggcggcca tttttacggc 1020
   gggcatatcg cctttgcgat ggactcctta aaaaatattg tggccaatat cgccgatctg 1080
   attgatcgcc aaatggcatt agtgatggac cctaaattta acaacggctt acccgctaac 1140
   ctttcgggtt caaccgggcc acgccgcgcc atcaaccatg gctttaaggc ggtgcaaatc 1200
   ggcgtttcag cctggacggc agaagcactg aaacacacta tgcccgcaag cgttttctca 1260
   cgctcaaccg aatgccacaa ccaagataaa gtcagcatgg gcaccattgc cgcccgcgac 1320
   tgtatgcgtg tattgcaact aacggaacaa gtcgccgccg cggcgctact tgctatgact 1380
   caaggcattg atctgcgtat cacacaaaac gagttagacg aagcctcact gacgccatca 1440
   ctggcgacca cgctcgccca agtgcgcgct gactttgagc cattagtcga agacagaccg 1500
   ctcgaagccg tgctacgcca aaccgtcgct aaaatccaag ccggtgaatg ggaagtgtgc 1560
   cgatga 1566
<210> 2
   <211> 521
   <212> PRT
   <213> Shewanella oneidensis
<400> 2
   Met Ser His Val Val Thr Gln Thr Thr Thr Val Glu Ser Pro Ile Glu
   1               5                   10                  15
   Phe Gly Arg Gln Leu Leu Thr Leu Glu Gln Val Val Ala Val Ala Lys
               20                  25                  30
   Gly Ala Lys Val Lys Leu Cys Asp Asp Ala Asp Tyr Gln Glu Tyr Ile
           35                  40                  45
   Gln Lys Gly Ala Arg Phe Ile Asp Ser Leu Leu His Glu Glu Gly Val
       50                  55                  60
   Val Tyr Gly Val Thr Thr Gly Tyr Gly Asp Ser Cys Thr Val Asn Val
   65                  70                  75                  80
   Ser Leu Asp Leu Val His Glu Leu Pro Leu His Leu Ser Arg Phe His
                   85                  90                  95
   Gly Cys Gly Leu Gly Glu Val Leu Ser Val Met Gln Ala Arg Ala Val
               100                 105                 110
   Met Ala Cys Arg Leu Asn Ser Leu Ala Ile Gly Lys Ser Gly Val Thr
           115                 120                 125
   Tyr Glu Leu Leu Lys Arg Ile Gln Thr Leu Leu Asn Leu Asn Ile Val
       130                 135                 140
   Pro Val Ile Pro Glu Glu Gly Ser Val Gly Ala Ser Gly Asp Leu Thr
   145                 150                 155                 160
   Pro Leu Ser Tyr Leu Ala Ala Val Leu Val Gly Glu Arg Glu Val Ile
                   165                 170                 175
   Tyr Gln Gly Glu Arg Arg Ala Thr Lys Glu Val Tyr His Glu Leu Asn
               180                 185                 190
   Ile Thr Pro His Val Leu Arg Pro Lys Glu Gly Leu Ala Leu Met Asn
           195                 200                 205
   Gly Thr Ala Val Met Thr Ala Leu Ala Cys Leu Ala Phe Asp Arg Ala
   210 215 220
   Gln Tyr Leu Ala Arg Leu Ala Ser Arg Ile Thr Ala Met Ala Ser Leu
   225 230 235 240
   Thr Leu Lys Gly Asn Ser Asn His Phe Asp Asp Ile Leu Phe Ala Ala
   245 250 255
   Lys Pro His Pro Gly Gln Asn Gln Ile Ala Thr Trp Ile Arg Glu Asp
   260 265 270
   Leu Asn His His Val His Pro Arg Asn Ser Asp Arg Leu Gln Asp Arg
   275 280 285
   Tyr Ser Ile Arg Cys Ala Pro His Ile Ile Gly Val Leu Gln Asp Ala
   290 295 300
   Leu Pro Phe Met Arg Gln Phe Ile Glu Thr Glu Val Asn Ser Ala Asn
   305 310 315 320
   Asp Asn Pro Ile Val Asp Ala Glu Gly Glu His Ile Leu His Gly Gly
   325 330 335
   His Phe Tyr Gly Gly His Ile Ala Phe Ala Met Asp Ser Leu Lys Asn
   340 345 350
   Ile Val Ala Asn Ile Ala Asp Leu Ile Asp Arg Gln Met Ala Leu Val
   355 360 365
   Met Asp Pro Lys Phe Asn Asn Gly Leu Pro Ala Asn Leu Ser Gly Ser
   370 375 380
   Thr Gly Pro Arg Arg Ala Ile Asn His Gly Phe Lys Ala Val Gln Ile
   385 390 395 400
   Gly Val Ser Ala Trp Thr Ala Glu Ala Leu Lys His Thr Met Pro Ala
   405 410 415
   Ser Val Phe Ser Arg Ser Thr Glu Cys His Asn Gln Asp Lys Val Ser
   420 425 430
   Met Gly Thr Ile Ala Ala Arg Asp Cys Met Arg Val Leu Gln Leu Thr
   435 440 445
   Glu Gln Val Ala Ala Ala Ala Leu Leu Ala Met Thr Gln Gly Ile Asp
   450 455 460
   Leu Arg Ile Thr Gln Asn Glu Leu Asp Glu Ala Ser Leu Thr Pro Ser
   465 470 475 480
   Leu Ala Thr Thr Leu Ala Gln Val Arg Ala Asp Phe Glu Pro Leu Val
   485 490 495
   Glu Asp Arg Pro Leu Glu Ala Val Leu Arg Gln Thr Val Ala Lys Ile
   500 505 510
   Gln Ala Gly Glu Trp Glu Val Cys Arg
   515 520
<210> 3
   <211> 1548
   <212> DNA
   <213> Idiomarina loihiensis
<400> 3
   atgacgacat cgattatagc ctttggtggc cgcccgttga ccattaaaga cgtggtagaa    60
   atagccaatg gcaaagcgca gatcgcgtta agcgacgcgc cggagtttgt cgaaaaagtc   120
   gatgccgggg ttcgttttct ggatgagctg ttagagcagg acggcgtgat ttatggcgtg   180
   acaacggggt acggagacag ctgcacagta tcggtaccgg agaacctggt taacgagttg   240
   cccattcatc ttacgcgttt tcatggttgt ggcctgggtg acacctttga tgaacaggaa   300
   acccgcgcta ttctggcaac ccgcttaagc tcgttggcac aaggttattc cggtgtcagc   360
   tgggagctgt tggaacgcct ggtggtgatg ttgaatgaaa acatgctgcc gcttattcca   420
   aaagagggca gtgttggtgc cagtggtgat cttacgcctt tgtcgtacat tgccggcgcg   480
   cttattggtg agcgtgacgt gcgcttccgg aataaagtga tgaacagcgc agaagccttt   540
   gagctgcttg gtatgtcggt tcacaaactg cggccaaaag aaggcctggc cattatgaac   600
   ggtaccgccg tgatgacggc tttggcttgc cttgcctggg accgggcgga gtacttaacc   660
   cggttatcca gccgcattac ctccttggcg agcatagctc tggaaggcaa cagtaaccat   720
   tttgatgacc tgctgtttgc cgtgaaaccc cataaagggc agcaacaggt cgccagctgg   780
   atccaacaag atctgaacca tgtggagcat ccgaggaatt catcgcgtct gcaggaccgg   840
   tactccatcc gctgcgcgcc tcatgttatc ggcgtactta aagacagttt accgtggttt   900
   aaagaaacca tagaaaatga attaaacagc gccaatgaca accccattat tgatggctta   960
   ggtgagcatg ttctgcacgg cgggcacttc tacggcggcc acattgctat ggtgatggac  1020
   tcgatgaaaa ctgctgtggc gaacctggcg gatttacacg accggcaaat ggcgttgttg  1080
   atggacacaa agatgaacca cggtttgccc tcgaacttgt ctgcggcaga aggtgagcgt  1140
   aagtctatta accacggctt taaagcggtg cagattggtt gctcagcctg gactgcggaa  1200
   gcattaaaac tgactatgcc agccagtgtc ttttcgcgct caaccgagtg tcataaccag  1260
   gacaaagtga gcatgggtac tattgctgcc cgtgactgta ttcgtattct ggaactgacc  1320
   gaacaggtgg ttgttgccac tttgctggca gcttatcaag gtgttgaact gcgtaagcgt  1380
   attacagaca aaccggtgaa cccatcggcc ggtgtcttag aaatgcttcg aagtctggct  1440
   gatcagtttg aactgctgac agaagaccgc cagttggagc cggaattacg ttattggctt  1500
   acccagttgc ggttaaaagt atggaatttg tatcaggagc cagcatga                1548
<210> 4
   <211> 515
   <212> PRT
   <213> Idiomarina loihiensis
<400> 4
   Met Thr Thr Ser Ile Ile Ala Phe Gly Gly Arg Pro Leu Thr Ile Lys
   1               5                   10                  15
   Asp Val Val Glu Ile Ala Asn Gly Lys Ala Gln Ile Ala Leu Ser Asp
               20                  25                  30
   Ala Pro Glu Phe Val Glu Lys Val Asp Ala Gly Val Arg Phe Leu Asp
           35                  40                  45
   Glu Leu Leu Glu Gln Asp Gly Val Ile Tyr Gly Val Thr Thr Gly Tyr
       50                  55                  60
   Gly Asp Ser Cys Thr Val Ser Val Pro Glu Asn Leu Val Asn Glu Leu
   65                  70                  75                  80
   Pro Ile His Leu Thr Arg Phe His Gly Cys Gly Leu Gly Asp Thr Phe
                   85                  90                  95
   Asp Glu Gln Glu Thr Arg Ala Ile Leu Ala Thr Arg Leu Ser Ser Leu
               100                 105                 110
   Ala Gln Gly Tyr Ser Gly Val Ser Trp Glu Leu Leu Glu Arg Leu Val
           115                 120                 125
   Val Met Leu Asn Glu Asn Met Leu Pro Leu Ile Pro Lys Glu Gly Ser
       130                 135                 140
   Val Gly Ala Ser Gly Asp Leu Thr Pro Leu Ser Tyr Ile Ala Gly Ala
   145                 150                 155                 160
   Leu Ile Gly Glu Arg Asp Val Arg Phe Arg Asn Lys Val Met Asn Ser
                   165                 170                 175
   Ala Glu Ala Phe Glu Leu Leu Gly Met Ser Val His Lys Leu Arg Pro
               180                 185                 190
   Lys Glu Gly Leu Ala Ile Met Asn Gly Thr Ala Val Met Thr Ala Leu
           195                 200                 205
   Ala Cys Leu Ala Trp Asp Arg Ala Glu Tyr Leu Thr Arg Leu Ser Ser
       210                 215                 220
   Arg Ile Thr Ser Leu Ala Ser Ile Ala Leu Glu Gly Asn Ser Asn His
   225                 230                 235                 240
   Phe Asp Asp Leu Leu Phe Ala Val Lys Pro His Lys Gly Gln Gln Gln
   245 250 255
   Val Ala Ser Trp Ile Gln Gln Asp Leu Asn His Val Glu His Pro Arg
   260 265 270
   Asn Ser Ser Arg Leu Gln Asp Arg Tyr Ser Ile Arg Cys Ala Pro His
   275 280 285
   Val Ile Gly Val Leu Lys Asp Ser Leu Pro Trp Phe Lys Glu Thr Ile
   290 295 300
   Glu Asn Glu Leu Asn Ser Ala Asn Asp Asn Pro Ile Ile Asp Gly Leu
   305 310 315 320
   Gly Glu His Val Leu His Gly Gly His Phe Tyr Gly Gly His Ile Ala
   325 330 335
   Met Val Met Asp Ser Met Lys Thr Ala Val Ala Asn Leu Ala Asp Leu
   340 345 350
   His Asp Arg Gln Met Ala Leu Leu Met Asp Thr Lys Met Asn His Gly
   355 360 365
   Leu Pro Ser Asn Leu Ser Ala Ala Glu Gly Glu Arg Lys Ser Ile Asn
   370 375 380
   His Gly Phe Lys Ala Val Gln Ile Gly Cys Ser Ala Trp Thr Ala Glu
   385 390 395 400
   Ala Leu Lys Leu Thr Met Pro Ala Ser Val Phe Ser Arg Ser Thr Glu
   405 410 415
   Cys His Asn Gln Asp Lys Val Ser Met Gly Thr Ile Ala Ala Arg Asp
   420 425 430
   Cys Ile Arg Ile Leu Glu Leu Thr Glu Gln Val Val Val Ala Thr Leu
   435 440 445
   Leu Ala Ala Tyr Gln Gly Val Glu Leu Arg Lys Arg Ile Thr Asp Lys
   450 455 460
   Pro Val Asn Pro Ser Ala Gly Val Leu Glu Met Leu Arg Ser Leu Ala
   465 470 475 480
   Asp Gln Phe Glu Leu Leu Thr Glu Asp Arg Gln Leu Glu Pro Glu Leu
   485 490 495
   Arg Tyr Trp Leu Thr Gln Leu Arg Leu Lys Val Trp Asn Leu Tyr Gln
   500 505 510
   Glu Pro Ala
   515
<210> 5
   <211> 1527
   <212> DNA
   <213> Fibrobacter succinogenes
<400> 5
   atgaaaaccg tcgttattgg aagtggtaag ctttcgattg aagatgttgt tgcgattgcg     60
   aaaaagcaag tcgcagtgga acttgatagt tctgcggaat tccagagaaa gattgattct    120
   ggtgcagaat tcttggacga agcgcttgca gaacatggtg gcatttatgg cgtgacaacg    180
   ggatatggtg attcctgcac gcaggtgttg ccgccggaac attacagcca gctcccgatt    240
   aacttgacgc gtttccatgg atgcggcctc ggtgattact ttgatgccga gacgacccgc    300
   gcgattatgg cggtacgctt gaatacgctt gcgcaaggtt tctctggtgt gagctatgca    360
   cttttgaaaa tcattacgca gtttttacag aacgatattt taccgctcat tccgcaggaa    420
   ggttccgtgg gcgcaagtgg cgacttgact ccgctttcgt atttggcagg tgccgtgatt    480
   ggtgaacgca atgtgatgta caagggcgaa cgtcgttctt ctttggacgt gatgaaggaa    540
   ttgggaatta ctccgcacaa gtttcgcccg aaagaagcaa ttgccattat gaacggcacg    600
   gcggcgatga atgcggtcgc ttgcatggct tattcccgtg cagaatacct ttctgacctc    660
   gcttgccgaa ttacagcgat gattacggtt gcaatgaagg gcaatgcgta tcactattat    720
   gaacgcttgt tcgaagcaaa accgcatccg ggacttggct tggctgcaaa gaaaattcgt    780
   gaagccttga atctcgatgt ggcgaagaat gtggttcccg agaaaatcca ggatccgtat    840
   tctctgcgtt gcgctccgca tgtgattgga cttttctatg attctagcgc gttcttgcgc    900
   caattgattg aaattgaact caacagcgct aatgacaatc cgattgtcga tccgttcaca    960
   aagaatattt tccacggtgg gcatttctat ggcgggcata tttgccttgc gatggatacg   1020
   ctcaagaaca tggttgcaaa cattgcggac cttttggacc gtcagctggc gatgattgtc   1080
   gatatcaagt tcaaccgtaa cttgccaccg aatcttgcgg ggagcaaggg cgattacgat   1140
   atcaatcatg gctttaaggc ggtgcagata ggcgtttctg catggacggc tgaagctttg   1200
   catttgacaa tgccgatgag cgtgttcagc cgttcgacgg aatgccacaa ccaagacaag   1260
   gtcagcatgg gtacgattgc ggcccgcgac tgcatccgcg tcttggagct ttcggaacag   1320
   gtggcagcgg caacgcttct ggcttcggct caggcacttc gcatacgctt ggaacgcaat   1380
   gaaatttcgg aagaccgcct caagaatctt caagagactt acaatcaggt attctcgaag   1440
   tttgctccgc ttgaatgtga ccgtcaattg gaacaggatt tgcgcaatac gctggagctg   1500
   attcgccaga aattttacgc tgtataa                                       1527
<210> 6
   <211> 508
   <212> PRT
   <213> Fibrobacter succinogenes
<400> 6
   Met Lys Thr Val Val Ile Gly Ser Gly Lys Leu Ser Ile Glu Asp Val
   1               5                   10                  15
   Val Ala Ile Ala Lys Lys Gln Val Ala Val Glu Leu Asp Ser Ser Ala
               20                  25                  30
   Glu Phe Gln Arg Lys Ile Asp Ser Gly Ala Glu Phe Leu Asp Glu Ala
           35                  40                  45
   Leu Ala Glu His Gly Gly Ile Tyr Gly Val Thr Thr Gly Tyr Gly Asp
       50                  55                  60
   Ser Cys Thr Gln Val Leu Pro Pro Glu His Tyr Ser Gln Leu Pro Ile
   65                  70                  75                  80
   Asn Leu Thr Arg Phe His Gly Cys Gly Leu Gly Asp Tyr Phe Asp Ala
                   85                  90                  95
   Glu Thr Thr Arg Ala Ile Met Ala Val Arg Leu Asn Thr Leu Ala Gln
               100                 105                 110
   Gly Phe Ser Gly Val Ser Tyr Ala Leu Leu Lys Ile Ile Thr Gln Phe
           115                 120                 125
   Leu Gln Asn Asp Ile Leu Pro Leu Ile Pro Gln Glu Gly Ser Val Gly
       130                 135                 140
   Ala Ser Gly Asp Leu Thr Pro Leu Ser Tyr Leu Ala Gly Ala Val Ile
   145                 150                 155                 160
   Gly Glu Arg Asn Val Met Tyr Lys Gly Glu Arg Arg Ser Ser Leu Asp
                   165                 170                 175
   Val Met Lys Glu Leu Gly Ile Thr Pro His Lys Phe Arg Pro Lys Glu
               180                 185                 190
   Ala Ile Ala Ile Met Asn Gly Thr Ala Ala Met Asn Ala Val Ala Cys
           195                 200                 205
   Met Ala Tyr Ser Arg Ala Glu Tyr Leu Ser Asp Leu Ala Cys Arg Ile
       210                 215                 220
   Thr Ala Met Ile Thr Val Ala Met Lys Gly Asn Ala Tyr His Tyr Tyr
   225                 230                 235                 240
   Glu Arg Leu Phe Glu Ala Lys Pro His Pro Gly Leu Gly Leu Ala Ala
                   245                 250                 255
   Lys Lys Ile Arg Glu Ala Leu Asn Leu Asp Val Ala Lys Asn Val Val
   260 265 270
   Pro Glu Lys Ile Gln Asp Pro Tyr Ser Leu Arg Cys Ala Pro His Val
   275 280 285
   Ile Gly Leu Phe Tyr Asp Ser Ser Ala Phe Leu Arg Gln Leu Ile Glu
   290 295 300
   Ile Glu Leu Asn Ser Ala Asn Asp Asn Pro Ile Val Asp Pro Phe Thr
   305 310 315 320
   Lys Asn Ile Phe His Gly Gly His Phe Tyr Gly Gly His Ile Cys Leu
   325 330 335
   Ala Met Asp Thr Leu Lys Asn Met Val Ala Asn Ile Ala Asp Leu Leu
   340 345 350
   Asp Arg Gln Leu Ala Met Ile Val Asp Ile Lys Phe Asn Arg Asn Leu
   355 360 365
   Pro Pro Asn Leu Ala Gly Ser Lys Gly Asp Tyr Asp Ile Asn His Gly
   370 375 380
   Phe Lys Ala Val Gln Ile Gly Val Ser Ala Trp Thr Ala Glu Ala Leu
   385 390 395 400
   His Leu Thr Met Pro Met Ser Val Phe Ser Arg Ser Thr Glu Cys His
   405 410 415
   Asn Gln Asp Lys Val Ser Met Gly Thr Ile Ala Ala Arg Asp Cys Ile
   420 425 430
   Arg Val Leu Glu Leu Ser Glu Gln Val Ala Ala Ala Thr Leu Leu Ala
   435 440 445
   Ser Ala Gln Ala Leu Arg Ile Arg Leu Glu Arg Asn Glu Ile Ser Glu
   450 455 460
   Asp Arg Leu Lys Asn Leu Gln Glu Thr Tyr Asn Gln Val Phe Ser Lys
   465 470 475 480
   Phe Ala Pro Leu Glu Cys Asp Arg Gln Leu Glu Gln Asp Leu Arg Asn
   485 490 495
   Thr Leu Glu Leu Ile Arg Gln Lys Phe Tyr Ala Val
   500 505
<210> 7
   <211> 1539
   <212> DNA
   <213> Vibrio vulnificus
<400> 7
   atgcaagcag aaaataatat tacctttggc caaggcagac tgacgattga ggatgttgcg     60
   gcgatcgctc aaggcgcaaa ggccacgtta aataactcag ttgaatttac cgccaaaatc    120
   gaccgcggtg tcgcctttct agaacgcctt cttaaagaag aaggggtgat ctatggcgtc    180
   accacaggat acggcgactc ttgtacagtc gcgataccgc ctcaattggt tgaagaacta    240
   ccacttcacc tcacgcgctt tcacggctgt ggcttaggca aaattctcac ccacgagcaa    300
   gcgcgcgctg tacttgctac acgtctgtgt tctttatcac aaggcgtttc tggcgtaagt    360
   catgacttac tcaatcagat cgttactttg attaatcacg atatttcacc ccgcattcct    420
   gaagaaggtt cggtgggagc cagtggtgat ctcactcccc tttcctacct tgctgcggcg    480
   ctggttggtg agcgagaagt gatctaccaa ggtgaagagc gtgcaaccgc tgaagtctac    540
   gccgaacttg gcatccaacc gattaaactg cgtcctaaag agggcttggc gctcatgaac    600
   ggcacatccg tgatgaccgc gttagcttgc cttgcctaca aacgcgcaga gtatctagct    660
   cagctcagca ccaaaatcac cgcgatggtc tctgtggcca tgcacggtaa tgacttccat    720
   tttgatgaag cgctctttgc agtcaaacct catccaggcc aacaacagat cgccgcttgg    780
   ttgcgtgatg acctgaaagc cgataagccg ccacgcaaca gtgaccgtct gcaagatcgt    840
   tactcactgc gctgtgcgcc gcacgtgatt ggtgttgtcc aagattcact accttggctg    900
   cgccaaatga tcgaaaacga gctcaacagt gccaatgaca acccaatcat cgatggtgat    960
   aacgaacgtg ttttacacgg cggtcacttc tatggtggtc acattgccat ggcaatggac   1020
   acactgaaaa ccggtattgc caacctcgcg gatctgctcg atcgtcaaat ggcgcaattg   1080
   atggattata agttcaacaa tggcttacca tttaacttga gcggcgccga aggggaacgc   1140
   aagcccatca accatggttt taaagcggtg caaattggca tttctgcttg gaccgcagaa   1200
   gcgctcaagc acaccatgcc tgccagcgta ttctcgcgtt caaccgagtg tcacaaccaa   1260
   gataaagtga gcatggggac tatcgctgcc cgcgattgtt tgcgagtatt ggaactgact   1320
   gaacaggtgg cggccgcgtc tctacttgct gcaacacaag ggatcgaaat tcgccgccgt   1380
   cgtggtgaac tggacgaaaa tcacatgagc gatcgtctga aagccatctg cagctcggta   1440
   ctcaatgagt ttgaatttgt cactgaagat cgcccattgg aaaaagatct gcgccagttc   1500
   atcactcaca ttcaacagcg tcattggtca ctgtattaa                          1539
<210> 8
   <211> 512
   <212> PRT
   <213> Vibrio vulnificus
<400> 8
   Met Gln Ala Glu Asn Asn Ile Thr Phe Gly Gln Gly Arg Leu Thr Ile
   1               5                   10                  15
   Glu Asp Val Ala Ala Ile Ala Gln Gly Ala Lys Ala Thr Leu Asn Asn
               20                  25                  30
   Ser Val Glu Phe Thr Ala Lys Ile Asp Arg Gly Val Ala Phe Leu Glu
   35 40 45
   Arg Leu Leu Lys Glu Glu Gly Val Ile Tyr Gly Val Thr Thr Gly Tyr
   50 55 60
   Gly Asp Ser Cys Thr Val Ala Ile Pro Pro Gln Leu Val Glu Glu Leu
   65 70 75 80
   Pro Leu His Leu Thr Arg Phe His Gly Cys Gly Leu Gly Lys Ile Leu
   85 90 95
   Thr His Glu Gln Ala Arg Ala Val Leu Ala Thr Arg Leu Cys Ser Leu
   100 105 110
   Ser Gln Gly Val Ser Gly Val Ser His Asp Leu Leu Asn Gln Ile Val
   115 120 125
   Thr Leu Ile Asn His Asp Ile Ser Pro Arg Ile Pro Glu Glu Gly Ser
   130 135 140
   Val Gly Ala Ser Gly Asp Leu Thr Pro Leu Ser Tyr Leu Ala Ala Ala
   145 150 155 160
   Leu Val Gly Glu Arg Glu Val Ile Tyr Gln Gly Glu Glu Arg Ala Thr
   165 170 175
   Ala Glu Val Tyr Ala Glu Leu Gly Ile Gln Pro Ile Lys Leu Arg Pro
   180 185 190
   Lys Glu Gly Leu Ala Leu Met Asn Gly Thr Ser Val Met Thr Ala Leu
   195 200 205
   Ala Cys Leu Ala Tyr Lys Arg Ala Glu Tyr Leu Ala Gln Leu Ser Thr
   210 215 220
   Lys Ile Thr Ala Met Val Ser Val Ala Met His Gly Asn Asp Phe His
   225 230 235 240
   Phe Asp Glu Ala Leu Phe Ala Val Lys Pro His Pro Gly Gln Gln Gln
   245 250 255
   Ile Ala Ala Trp Leu Arg Asp Asp Leu Lys Ala Asp Lys Pro Pro Arg
   260 265 270
   Asn Ser Asp Arg Leu Gln Asp Arg Tyr Ser Leu Arg Cys Ala Pro His
   275 280 285
   Val Ile Gly Val Val Gln Asp Ser Leu Pro Trp Leu Arg Gln Met Ile
   290 295 300
   Glu Asn Glu Leu Asn Ser Ala Asn Asp Asn Pro Ile Ile Asp Gly Asp
   305                 310                 315                 320
   Asn Glu Arg Val Leu His Gly Gly His Phe Tyr Gly Gly His Ile Ala
                   325                 330                 335
   Met Ala Met Asp Thr Leu Lys Thr Gly Ile Ala Asn Leu Ala Asp Leu
               340                 345                 350
   Leu Asp Arg Gln Met Ala Gln Leu Met Asp Tyr Lys Phe Asn Asn Gly
           355                 360                 365
   Leu Pro Phe Asn Leu Ser Gly Ala Glu Gly Glu Arg Lys Pro Ile Asn
       370                 375                 380
   His Gly Phe Lys Ala Val Gln Ile Gly Ile Ser Ala Trp Thr Ala Glu
   385                 390                 395                 400
   Ala Leu Lys His Thr Met Pro Ala Ser Val Phe Ser Arg Ser Thr Glu
                   405                 410                 415
   Cys His Asn Gln Asp Lys Val Ser Met Gly Thr Ile Ala Ala Arg Asp
               420                 425                 430
   Cys Leu Arg Val Leu Glu Leu Thr Glu Gln Val Ala Ala Ala Ser Leu
           435                 440                 445
   Leu Ala Ala Thr Gln Gly Ile Glu Ile Arg Arg Arg Arg Gly Glu Leu
       450                 455                 460
   Asp Glu Asn His Met Ser Asp Arg Leu Lys Ala Ile Cys Ser Ser Val
   465                 470                 475                 480
   Leu Asn Glu Phe Glu Phe Val Thr Glu Asp Arg Pro Leu Glu Lys Asp
                   485                 490                 495
   Leu Arg Gln Phe Ile Thr His Ile Gln Gln Arg His Trp Ser Leu Tyr
               500                 505                 510
<210> 9
   <211> 2151
   <212> DNA
   <213> Rhodotorula glutinis
<400> 9
   atggcgccga gcctggatag cattagccat agctttgcga acggcgtggc gagcgcgaaa    60
   caggcggtga acggcgcgag caccaacctg gcggtggcgg gcagccatct gccgaccacc   120
   caggtgaccc aggtggatat tgtggaaaaa atgctggcgg cgccgaccga tagcaccctg   180
   gaactggatg gctatagcct gaacctgggc gatgtggtga gcgcggcgcg taaaggccgt   240
   ccggtgcgtg tgaaagatag cgatgaaatt cgtagcaaaa ttgataaaag cgtggaattt   300
   ctgcgtagcc agctgagcat gagcgtgtat ggcgtgacca ccggctttgg cggtagcgcg    360
   gatacccgta ccgaagatgc gattagcctg cagaaagcgc tgctggaaca tcagctgtgc    420
   ggcgtgctgc cgagcagctt tgatagcttt cgtctgggcc gtggcctgga aaacagcctg    480
   ccgctggaag tggtgcgtgg cgcgatgacc attcgtgtga acagcctgac ccgtggccat    540
   agcgcggtgc gtctggtggt gctggaagcg ctgaccaact ttctgaacca tggcattacc    600
   ccgattgtgc cgctgcgtgg caccattagc gcgagcggcg atctgagccc gctgagctat    660
   attgcggcgg cgattagcgg ccatccggat agcaaagtgc atgtggtgca tgaaggcaaa    720
   gaaaaaattc tgtatgcgcg tgaagcgatg gcgctgttta acctggaacc ggtggtgctg    780
   ggtccgaaag aaggcctggg cctggtgaac ggcaccgcgg tgagcgcgag catggcgacc    840
   ctggcgctgc atgatgcgca tatgctgagc ctgctgagcc agagcctgac cgcgatgacc    900
   gtggaagcga tggtgggcca tgcgggcagc tttcatccgt ttctgcatga tgtgacccgt    960
   ccgcatccga cccagattga agtggcgggc aacattcgta aactgctgga aggcagccgt   1020
   tttgcggtgc atcatgaaga agaagtgaaa gtgaaagatg atgaaggcat tctgcgtcag   1080
   gatcgttatc cgctgcgtac cagcccgcag tggctgggcc cgctggtgag cgatctgatt   1140
   catgcgcatg cggtgctgac cattgaagcg ggtcagagca ccaccgataa cccgctgatt   1200
   gatgtggaaa acaaaaccag ccatcatggc ggcaactttc aggcggcggc ggtggcgaac   1260
   accatggaaa aaacccgtct gggcctggcg cagattggca aactgaactt tacccagctg   1320
   accgaaatgc tgaacgcggg catgaaccgt ggcctgccga gctgcctggc ggcggaagat   1380
   ccgagcctga gctatcattg caaaggcctg gatattgcgg cggcggcgta taccagcgaa   1440
   ctgggccatc tggcgaaccc ggtgaccacc catgtgcagc cggcggaaat ggcgaaccag   1500
   gcggtgaaca gcctggcgct gattagcgcg cgtcgtacca ccgaaagcaa cgatgtgctg   1560
   agcctgctgc tggcgaccca tctgtattgc gtgctgcagg cgattgatct gcgtgcgatt   1620
   gaatttgaat ttaaaaaaca gtttggcccg gcgattgtga gcctgattga tcagcatttt   1680
   ggcagcgcga tgaccggcag caacctgcgt gatgaactgg tggaaaaagt gaacaaaacc   1740
   ctggcgaaac gtctggaaca gaccaacagc tatgatctgg tgccgcgttg gcatgatgcg   1800
   tttagctttg cggcgggcac cgtggtggaa gtgctgagca gcaccagcct gagcctggcg   1860
   gcggtgaacg cctggaaagt ggcggcggcg gaaagcgcga ttagcctgac ccgtcaggtg   1920
   cgtgaaacct tttggagcgc ggcgagcacc agcagcccgg cgctgagcta tctgagcccg   1980
   cgtacccaga ttctgtatgc gtttgtgcgt gaagaactgg gcgtgaaagc gcgtcgtggc   2040
   gatgtgtttc tgggcaaaca ggaagtgacc attggcagca acgtgagcaa aatttatgaa   2100
   gcgattaaaa gcggccgtat taacaacgtg ctgctgaaaa tgctggcgta a             2151
<210> 10
   <211> 716
   <212> PRT
   <213> Rhodotorula glutinis
<400> 10
   Met Ala Pro Ser Leu Asp Ser Ile Ser His Ser Phe Ala Asn Gly Val
   1               5                   10                  15
   Ala Ser Ala Lys Gln Ala Val Asn Gly Ala Ser Thr Asn Leu Ala Val
               20                  25                  30
   Ala Gly Ser His Leu Pro Thr Thr Gln Val Thr Gln Val Asp Ile Val
           35                  40                  45
   Glu Lys Met Leu Ala Ala Pro Thr Asp Ser Thr Leu Glu Leu Asp Gly
       50                  55                  60
   Tyr Ser Leu Asn Leu Gly Asp Val Val Ser Ala Ala Arg Lys Gly Arg
   65                  70                  75                  80
   Pro Val Arg Val Lys Asp Ser Asp Glu Ile Arg Ser Lys Ile Asp Lys
                   85                  90                  95
   Ser Val Glu Phe Leu Arg Ser Gln Leu Ser Met Ser Val Tyr Gly Val
               100                 105                 110
   Thr Thr Gly Phe Gly Gly Ser Ala Asp Thr Arg Thr Glu Asp Ala Ile
           115                 120                 125
   Ser Leu Gln Lys Ala Leu Leu Glu His Gln Leu Cys Gly Val Leu Pro
       130                 135                 140
   Ser Ser Phe Asp Ser Phe Arg Leu Gly Arg Gly Leu Glu Asn Ser Leu
   145                 150                 155                 160
   Pro Leu Glu Val Val Arg Gly Ala Met Thr Ile Arg Val Asn Ser Leu
                   165                 170                 175
   Thr Arg Gly His Ser Ala Val Arg Leu Val Val Leu Glu Ala Leu Thr
               180                 185                 190
   Asn Phe Leu Asn His Gly Ile Thr Pro Ile Val Pro Leu Arg Gly Thr
           195                 200                 205
   Ile Ser Ala Ser Gly Asp Leu Ser Pro Leu Ser Tyr Ile Ala Ala Ala
       210                 215                 220
   Ile Ser Gly His Pro Asp Ser Lys Val His Val Val His Glu Gly Lys
   225                 230                 235                 240
   Glu Lys Ile Leu Tyr Ala Arg Glu Ala Met Ala Leu Phe Asn Leu Glu
                   245                 250                 255
   Pro Val Val Leu Gly Pro Lys Glu Gly Leu Gly Leu Val Asn Gly Thr
   260 265 270
   Ala Val Ser Ala Ser Met Ala Thr Leu Ala Leu His Asp Ala His Met
   275 280 285
   Leu Ser Leu Leu Ser Gln Ser Leu Thr Ala Met Thr Val Glu Ala Met
   290 295 300
   Val Gly His Ala Gly Ser Phe His Pro Phe Leu His Asp Val Thr Arg
   305 310 315 320
   Pro His Pro Thr Gln Ile Glu Val Ala Gly Asn Ile Arg Lys Leu Leu
   325 330 335
   Glu Gly Ser Arg Phe Ala Val His His Glu Glu Glu Val Lys Val Lys
   340 345 350
   Asp Asp Glu Gly Ile Leu Arg Gln Asp Arg Tyr Pro Leu Arg Thr Ser
   355 360 365
   Pro Gln Trp Leu Gly Pro Leu Val Ser Asp Leu Ile His Ala His Ala
   370 375 380
   Val Leu Thr Ile Glu Ala Gly Gln Ser Thr Thr Asp Asn Pro Leu Ile
   385 390 395 400
   Asp Val Glu Asn Lys Thr Ser His His Gly Gly Asn Phe Gln Ala Ala
   405 410 415
   Ala Val Ala Asn Thr Met Glu Lys Thr Arg Leu Gly Leu Ala Gln Ile
   420 425 430
   Gly Lys Leu Asn Phe Thr Gln Leu Thr Glu Met Leu Asn Ala Gly Met
   435 440 445
   Asn Arg Gly Leu Pro Ser Cys Leu Ala Ala Glu Asp Pro Ser Leu Ser
   450 455 460
   Tyr His Cys Lys Gly Leu Asp Ile Ala Ala Ala Ala Tyr Thr Ser Glu
   465 470 475 480
   Leu Gly His Leu Ala Asn Pro Val Thr Thr His Val Gln Pro Ala Glu
   485 490 495
   Met Ala Asn Gln Ala Val Asn Ser Leu Ala Leu Ile Ser Ala Arg Arg
   500 505 510
   Thr Thr Glu Ser Asn Asp Val Leu Ser Leu Leu Leu Ala Thr His Leu
   515 520 525
   Tyr Cys Val Leu Gln Ala Ile Asp Leu Arg Ala Ile Glu Phe Glu Phe
   530 535 540
   Lys Lys Gln Phe Gly Pro Ala Ile Val Ser Leu Ile Asp Gln His Phe
   545 550 555 560
   Gly Ser Ala Met Thr Gly Ser Asn Leu Arg Asp Glu Leu Val Glu Lys
   565 570 575
   Val Asn Lys Thr Leu Ala Lys Arg Leu Glu Gln Thr Asn Ser Tyr Asp
   580 585 590
   Leu Val Pro Arg Trp His Asp Ala Phe Ser Phe Ala Ala Gly Thr Val
   595 600 605
   Val Glu Val Leu Ser Ser Thr Ser Leu Ser Leu Ala Ala Val Asn Ala
   610 615 620
   Trp Lys Val Ala Ala Ala Glu Ser Ala Ile Ser Leu Thr Arg Gln Val
   625 630 635 640
   Arg Glu Thr Phe Trp Ser Ala Ala Ser Thr Ser Ser Pro Ala Leu Ser
   645 650 655
   Tyr Leu Ser Pro Arg Thr Gln Ile Leu Tyr Ala Phe Val Arg Glu Glu
   660 665 670
   Leu Gly Val Lys Ala Arg Arg Gly Asp Val Phe Leu Gly Lys Gln Glu
   675 680 685
   Val Thr Ile Gly Ser Asn Val Ser Lys Ile Tyr Glu Ala Ile Lys Ser
   690 695 700
   Gly Arg Ile Asn Asn Val Leu Leu Lys Met Leu Ala
   705 710 715